# FASCICULE DE BREVET EUROPEEN

(11) **EP 2 512 494 B1**
(45) Date de publication et mention de la délivrance du brevet: **08.04.2015**
(21) Numéro de dépôt: 10801631.2
(22) Date de dépôt: 06.12.2010
(51) Int. Cl.: A61K 35/52, A61K 9/50

(54) **COMPOSITION DE MATERIEL SEMINAL PORCIN POUR L'INSEMINATION ARTIFICIELLE DE TRUIES**
PORCINE SAMENMATERIALZUSAMMENSETZUNG ZUR KÜNSTLICHEN BEFRUCHTUNG VON SÄUEN
PORCINE SEMINAL MATERIAL COMPOSITION FOR ARTIFICIAL INSEMINATION OF SOWS

(30) Priorité: 14.12.2009 FR 0958906
(43) Date de publication de la demande: 24.10.2012
(73) Titulaire: Genes Diffusion, 59500 Douai (FR)
(72) Inventeur: LIEGEOIS, Luc, F-59283 Raimbeaucourt (FR)
(74) Mandataire: Matkowska, Franck
(86) Numéro de dépôt international: PCT/FR2010/052620
(87) Numéro de publication internationale: WO 2011/080438

(56) Documents cités:
- EP-A2- 0 922 451
- WO-A2-2006/106400
- TORRE M L ET AL: "Controlled release of swine semen encapsulated in calcium alginate beads", BIOMATERIALS, ELSEVIER SCIENCE PUBLISHERS BV., BARKING, GB LNKD- DOI:10.1016/S0142-9612(00)00035-1, vol. 21, no. 14, 1 juillet 2000 (2000-07-01), pages 1493-1498, XP004199070, ISSN: 0142-9612
- TORRE M L ET AL: "Boar semen controlled delivery system: storage and in vitro spermatozoa release", JOURNAL OF CONTROLLED RELEASE, ELSEVIER, AMSTERDAM, NL LNKD- DOI:10.1016/S0168-3659(02)00290-0, vol. 85, no. 1-3, 13 décembre 2002 (2002-12-13), pages 83-89, XP004397768, ISSN: 0168-3659
- TORRE M L ET AL: "CALCIUM ALGINATE CAPSULES CONTAINING A HYDROPHILIC POLYMER FOR THE ENCAPSULATION OF SWINE SPERMATOZOA", SCIENCES TECHNIQUES ET PRATIQUES STP PHARMA SCIENCES, PARIS, FR, vol. 8, no. 4, 1 janvier 1998 (1998-01-01) , pages 233-236, XP009073273, ISSN: 1157-1489

## Description

### Objet de l'invention

La présente invention est relative à une composition contenant du matériel séminal porcin destinée à l'insémination artificielle de truies, ainsi que le procédé d'obtention de cette composition.

La présente invention est également relative à une trousse comportant un ou plusieurs flacon(s) avec ladite composition ou les éléments de ladite composition et un moyen d'insémination (sonde) apte à effectuer l'insémination artificielle d'une truie par cette composition.

### Arrière plan technologique et état de la technique à la base de l'invention

La technologie d'insémination artificielle est utilisée pour l'insémination de plusieurs espèces domestiques depuis de nombreuses années.

Pour l'insémination artificielle porcine, la plupart des femelles élevées sont conduites en « bandes » afin de regrouper les interventions et de les concentrer pour réduire le temps d'observation et d'opération sur les animaux. Physiologiquement, le sevrage de porcelets engendre une reprise du cycle de la truie qui revient en chaleur en moyenne cinq jours après le sevrage. Il suffit donc de sevrer ensemble les porcelets, généralement le mercredi et le jeudi, pour que 10% des truies commencent leur chaleur le dimanche, 50% le lundi, 30% le mardi et les derniers 10% le reste de la semaine. La durée moyenne de la période de chaleur s'étale de 24 à 72 heures. Les chaleurs des premières truies se manifestent en général plus longuement. Durant ces manifestations, l'éleveur peut inséminer selon ses habitudes toutes les 12 ou 24 heures avec de nombreux temps variables.

Afin de couvrir les femelles, les éleveurs utilisent en moyenne, par cycle, 2,6 doses contenant chacune 2,2 milliards de spermatozoïdes. Pour réduire le nombre d'interventions tout en assurant une bonne disponibilité du spermatozoïde dans le tractus génital de la femelle, des moyens ont dû être mis en oeuvre pour obtenir une insémination de longue durée.

Le concept d'encapsulation du sperme avec libération différée dans le temps a été décrit dans le brevet EP 0 922 451 et est basé sur l'utilisation de capsules comprenant un noyau liquide contenant une suspension de matériel séminal de porc ainsi qu'un polymère biodégradable et/ou biocompatible, le tout étant enrobé d'un film consistant en un alginate et d'un métal bi ou trivalent, éventuellement réticulé. Habituellement le film d'alginate de métaux bivalents est sélectionné parmi l'alginate de calcium, de strontium, et de zinc. Les alginates de métaux trivalents sont de préférence sélectionnés parmi ceux d'aluminium, de fer et de chrome.

Un grand nombre de polysaccharides tels que l'alginate, les chitosans, les pectines, les carraghénanes, les xanthanes ont la particularité de former des hydrogels de manière spontanée par modification physique ou chimique, par exemple par une modification du pH, de la température ou par addition d'un contre-ion approprié. L'alginate de sodium est par exemple un polysaccharide polyanionique naturel de la paroi cellulaire d'algue brune (telle que le Macrocystis pyrifera, le Laminaria digitata, l'Ascophyllum nodosum) qui représente jusqu'à 40% du poids sec de l'algue.

L'alginate est constitué d'un mélange d'acide manuronique et d'acide guluronique qui est soluble dans l'eau, mais forme un gel en présence de cations di- ou trivalent. Des composés comme l'EDTA ont pour action de capter également ces ions et de déstructurer les gels obtenus.

Le sperme porcin est très sensible au stress induit par un choc thermique brutal ainsi que par une variation de pression osmotique ou de pH et sa motilité se trouve vite altérée par des variations du milieu. Cette altération va se manifester par une évolution vers l'apoptose des spermatozoïdes qui suit le phénomène de capacitation. Ce phénomène se visualise par l'apparition d'agglutinats, ultime phase avant la mort des cellules.

### Buts de l'invention

La présente invention a pour but de fournir une nouvelle composition comportant du matériel séminal porcin destinée à une insémination artificielle de truies et qui ne présente pas les inconvénients de l'état de la technique.

La présente invention a également pour but de fournir un procédé d'obtention d'une telle composition, ainsi qu'une trousse comportant un ou plusieurs flacon(s) contenant ladite composition ou contenant les éléments de ladite composition et un moyen d'insémination (sonde) de cette composition à des truies.

Un but particulier de la présente invention vise à fournir une telle composition caractérisée par une toxicité réduite ou stress réduit) vis-à-vis des spermatozoïdes présents dans un matériel séminal (encapsulé et/ou libre) tout en permettant une administration efficace et prolongée desdits spermatozoïdes à une truie.

### Eléments caractéristiques de l'invention

Un premier objet de l'invention est relatif à une composition de matériel séminal porcin destiné à l'insémination artificielle de truies et comprenant des capsules incorporant du matériel séminal porcin, enrobé d'un film d'alginate qui ne présente pas les inconvénients de l'état de la technique.

Dans la composition de l'invention la concentration en spermatozoïdes du matériel séminal porcin présent dans les capsules est comprise entre 900 millions et 2000 millions de spermatozoïdes par millilitre.

En outre, la composition du matériel séminal porcin de l'invention peut comporter une fraction libre de matériel séminal porcin, c'est-à-dire une fraction libre de matériel séminal porcin non présente dans des capsules et qui n'est pas enrobée d'un film d'alginate (d'un ion bi ou trivalent). Dans ce cas, la concentration de spermatozoïdes présents dans la fraction libre du matériel séminal porcin comporte entre 25 millions et 35 millions de spermatozoïdes par millilitre.

Ceci signifie que la composition de l'invention comporte entre 1 et 3 milliards (de préférence entre 1,5 et 2,5 milliards) de spermatozoïdes encapsulés et/ou entre 1 et 2,5 milliards (de préférence entre 1,5 et 2 milliards) de spermatozoïdes libres, c'est-à-dire des spermatozoïdes non encapsulés.

De préférence, dans la composition de l'invention, l'ion choisi est du baryum, mais peut être également un autre ion métallique, de préférence bivalent, tel que du calcium ou un autre ion alcalino-terreux.

Un autre aspect de la présente invention est relatif à un procédé d'obtention de la composition de l'invention qui comprend essentiellement les étapes suivantes :
- un matériel séminal porcin récolté et soumis à une ou plusieurs étapes d'extraction de plus de 50%, de préférence de plus de 65%, encore de préférence de plus de 75% du plasma séminal présent initialement dans ce matériel séminal porcin et de récolte du matériel séminal porcin enrichi en spermatozoïdes suite à cette extraction.
- ledit matériel séminal porcin est ensuite enrichi en spermatozoïdes et additionné de 2% à 8% d'un ion bi ou trivalent, de préférence de baryum est mis en suspension et ajouté, goutte à goutte, à une solution d'alginate d'un ion métallique monovalent, de préférence de sodium pour former des capsules enrobant ledit matériel séminal enrichi en spermatozoïdes.
- lesdites capsules obtenues sont ensuite récoltées (pour former une fraction encapsulée de matériel séminal porcin) et sont de préférence mélangées avec une fraction libre de matériel séminal porcin.

Dans le procédé de l'invention, les étapes d'extraction du plasma séminal porcin comporte(nt) ou consiste(nt) en une ou plusieurs étape(s) de centrifugation du matériel séminal porcin suivie(s) d'une ou plusieurs étape(s) d'élimination du plasma séminal présent dans le surnageant du milieu soumis à la centrifugation et de récolte du matériel séminal porcin enrichi en spermatozoïdes.

Un autre objet de l'invention est relatif à une trousse d'insémination comportant un ou plusieurs flacon(s) contenant la composition ou les éléments de la composition de matériel séminal porcin selon l'invention, en particulier les fractions libres et/ou les fractions encapsulées ainsi qu'un moyen d'insémination apte à effectuer l'insémination artificielle d'une truie par cette composition et optionnellement un flacon contenant un dilueur de cette composition, en particulier un dilueur de la fraction libre de matériel séminal porcin, tel que le produit Gedil®.

### Description détaillée de l'invention

Les Inventeurs ont découvert de manière inattendue que l'encapsulation d'un matériel séminal porcin telle que décrite dans le brevet EP 0 922 451 induisait une « toxicité » (ou un « stress ») pour les spermatozoïdes porcins présents dans les capsules obtenues ou les spermatozoïdes libres (non encapsulés), mais mis en contact avec ces capsules.

En particulier, les inventeurs ont découvert de manière inattendue qu'au moins deux produits présents dans ces capsules sont susceptibles de générer cette « toxicité » (ou un « stress ») affectant les propriétés de spermatozoïdes porcins. Les Inventeurs ont démontré pour la première fois, qu'une solution de baryum, en particulier les ions baryum présents dans cette solution, ainsi que de l'alginate de sodium ou de l'alginate polymérisé présents dans ces capsules altèrent les propriétés essentielles (telle que la motilité) des spermatozoïdes porcins.

Ces effets néfastes sur les spermatozoïdes porcins sont démontrées dans les tableaux 1 et 2 qui présentent le résultat d'un test de « toxicité » (ou « stress ») obtenu par addition de chlorure de baryum (BaCl₂ à 5 ou 10%) sur une semence libre et montre après deux jours une réduction significative de la motilité des spermatozoïdes. Pour obtenir une fertilisation efficace, plus de 50% des spermatozoïdes doivent être « motiles » (c'est-à-dire présenter une vitesse de déplacement individuelle supérieure à 20 micromètres par seconde).

Tableau 1 : Effet du chlorure de Baryum (BaCl₂) à 5 ou 10% sur la motilité des spermatozoïdes présents dans la semence libre. Les deux valeurs (X-Y) mentionnées dans ce tableau sont le résultat de deux mesures :
La première valeur (X) sur 100 signifie que X spermatozoïdes sur 100 présentent une motilité supérieure à 20 micromètres par seconde (X spermatozoïdes dits « motiles »)
La seconde valeur (Y) sur 100 signifie que Y de ces mêmes 100 spermatozoïdes ont une même vitesse de déplacement supérieure ou égale à 80 micromètres par seconde (Y spermatozoïdes dits « progressifs »)

| **ECHANTILLONS** | **5 % BaCI2** | **10 % BaCl2** |
|---|---|---|
| Verrat 1 CG0533 | 68-19 | 58-12 |
| Verrat 2 CG6558 | 45-20 | 29-07 |
| Verrat 3 Lwc Derby | 40-10 | 24-05 |
| Verrat 4 DB2125 | 69-30 | 40-15 |
| Verrat 5 PF2182 | 50-26 | 48-28 |

**Tableau 2: Effet du chlorure de Baryum (BaCl₂) à 2% sur la motilité des spermatozoïdes présents dans la semence libre.**

| **ECHANTILLONS** | **TEMOIN GEDIL** | | **GEDIL + 2 % BaCl2** | |
|---|---|---|---|---|
| | **J+1** | **J+3** | **J+1** | **J+3** |
| Verrat 1 | 53-23 | 51-19 | 54-29 | 45-23 |
| Verrat 2 | 85-34 | 87-42 | 82-46 | 78-50 |
| Verrat 3 | 85-38 | 82-43 | 83-54 | 80-40 |
| Verrat 4 | 86-27 | 84-25 | 84-46 | 79-45 |
| Verrat 5 | 80-20 | 77-24 | 77-34 | 73-22 |
| Verrat 6 | 75-19 | 72-23 | 57-28 | 60-23 |
| Verrat 7 | 79-33 | 84-34 | 79-45 | 84-45 |
| Verrat 8 | 75-23 | 75-48 | 70-45 | 73-52 |
| Verrat 9 | 68-11 | 60-11 | 63-17 | 64-17 |
| Verrat 10 | 80-41 | 77-43 | Non interprétable | 60-34 |

Le tableau 3 montre l'effet du nombre de capsules (billes) ayant une taille comprise entre 50 µm et 8 mm, sur la motilité des spermatozoïdes après 1 et 3 jours. Le Gedil® est un dilueur constitué d'un milieu biologique favorable à la conservation des spermatozoïdes. Les inventeurs ont également dilué les spermatozoïdes dans d'autres milieux que le Gedil® et observent également une « toxicité » (ou un « stress ») induit par l'addition de BaCl₂.

**Tableau 3 : Effet du nombre de billes (capsules) sur la semence libre :**

| **ECHANTILLONS** | **TEMOIN GEDIL** | **GEDIL + 4 billes par tubes** | | **GEDIL + 20 billes par tube** | |
|---|---|---|---|---|---|
| | | **J+1** | **J+3** | **J+1** | **J+3** |
| Verrat 1 | 73-15 | 69-26 | 66-09 | 51-16 | 51-05 |
| Verrat 2 | 81-45 | 77-52 | 73-13 | 50-26 | 58-10 |
| Verrat 3 | 82-39 | 75-49 | 71-15 | 47-20 | 60-11 |
| Verrat 4 | 85-41 | 82-36 | 83-18 | 41-07 | 51-08 |
| Verrat 5 | 57-18 | 64-30 | 69-10 | 55-17 | 57-05 |
| Verrat 6 | 67-25 | 70-33 | 80-17 | 55-12 | 57-05 |
| Verrat 7 | 86-36 | 86-37 | 82-12 | 51-12 | 69-09 |
| Verrat 8 | 78-41 | 85-49 | 87-14 | 56-31 | 60-13 |
| Verrat 9 | 72-15 | 61-15 | 70-08 | 49-18 | 62-09 |
| Verrat 10 | 78-41 | 66-37 | 74-07 | 68-23 | 59-03 |

Remarque : La motilité des spermatozoïdes est affectée: les mouvements obtenus sont fluides avec l'addition de 4 billes (capsules) et les mouvements sont saccadés avec l'addition de 20 billes (capsules). Les inventeurs ont également dilué les spermatozoïdes dans d'autres milieux que le Gedil® et observent également une toxicité proportionnelle au nombre de billes (capsules) ajoutées.

Afin de réduire les effets nocifs de ces produits (toxiques), les inventeurs ont obtenu une réduction de la proportion globale de ces éléments toxiques dans la composition de l'invention, en réalisant une concentration du liquide séminal porcin et des spermatozoïdes présents dans ces capsules. Cette opération est obtenue en retirant une importante proportion du plasma séminal présent dans ce matériel séminal porcin à encapsuler. De ce fait, la concentration des spermatozoïdes porcins dans les capsules augmente de manière significative, ce qui conduit à réduire la quantité de ces capsules et des éléments (toxiques) issus de ces capsules dans la composition finale obtenue composée de spermatozoïdes encapsulés et de spermatozoïdes libres (c'est-à-dire de spermatozoïdes non encapsulés), et éventuellement de dilueur.

La présente invention sera décrite en détail dans les exemples ci-dessous présentés à titre d'illustration non limitative de l'invention.

### Exemple 1 :

Une semence porcine est récoltée et arrive au laboratoire à une température de l'ordre de 33°C à 35°C. Elle est mesurée pour sa concentration, son volume et sa couleur, puis introduite dans une centrifugeuse tournant à environ 800 g, pendant une durée d'environ 10 minutes. Par cette centrifugation, environ les 2/3 du surnageant (contenant du plasma séminal) sont retirés et mesurés en concentration par soustraction afin de connaître la quantité de spermatozoïdes restants, le 1/3 du volume conservé, contenant l'essentiel des spermatozoïdes, est destiné à être encapsulé.

La concentration en spermatozoïdes au sein du matériel séminal destiné à être encapsulé peut se faire également par d'autres techniques bien connues de l'homme de l'art, telles qu'une sédimentation (par exemple au moment d'un shift de température d'environ 17°C) ou par une filtration sur membrane (par exemple une membrane d'environ 0,1 µm) de manière à éliminer ou réduire une importante proportion (soit plus de 50% , de préférence plus de 65%, encore de préférence plus de 75% ou plus de 85%) du plasma séminal.

Cette fraction est préalablement mélangée avec une solution de baryum (addition d'une solution (aqueuse) saturée de chlorure de baryum -215g de chlorure de baryum en poudre par litre- afin d'obtenir une concentration finale en ions baryum de 25 mmol/L), puis injectée au goutte à goutte dans une solution d'alginate de sodium en agitation (alginate de sodium en solution ; 0,01% à 1% poids par volume). On peut injecter ainsi environ 500 ml de sperme additionné de chlorure de baryum dans un bain de 20 litres d'alginate de sodium.

Les capsules (billes) se forment instantanément au contact de la périphérie de la goutte et s'épaississent durant plusieurs dizaines de minutes. Le matériel spermatique encapsulé dans le film (gel) d'alginate de sodium, et comprenant plus de 900 millions de spermatozoïdes par millilitre, est ensuite séparé par simple filtration. Après lavage, les capsules (billes) sont récupérées dans un panier de séparation, puis quantifiées en volume. Ensuite, elles sont mélangées à une fraction de matériel séminal libre dilué (comprenant environ 33 millions de spermatozoïdes par millilitre). Ce mélange d'une fraction libre combinée à une fraction encapsulée permet de féconder une truie, si l'ovulation de la truie se situe dans les quelques heures suivantes et permet aussi d'obtenir la fécondation sur une plus longue période, grâce à la libération postposée des spermatozoïdes encapsulés plus tard. La composition obtenue est ensuite injectée dans une sonde, dont les orifices d'entrée et de sortie ont été adaptés au passage des capsules (diamètre de 0,8 cm environ).

Cette « fraction encapsulée » représente un volume de capsules contenant environ 1,5 à environ 2,5 milliards de spermatozoïdes.

Alternativement, la fraction encapsulée, qui représente un volume de billes capsules contenant de environ 1,5 à environ 2,5 milliards de spermatozoïdes est mélangée à la fraction libre, diluée et contenant entre environ 1,5 milliard et environ 2,5 milliards de spermatozoïdes présents dans un dilueur adapté. Ces 2 fractions sont globalement mélangées sous agitation.

L'ovulation a été introduite par sevrage sans addition d'hormones. La détection de chaleurs chez la truie est mesurée deux fois par jour dès le 3^{ème} jour après sevrage. L'insémination est pratiquée après détection de l'ovulation. Un éventuel état gestationnel est mesuré par échographie et l'ensemble des données sont mesurées. Dans l'ensemble, les inventeurs constatent une augmentation du nombre de gestations chez les truies fécondées par la composition de l'invention (où les spermatozoïdes ont été concentrés avant encapsulation) par rapport aux truies fécondées par une composition sans concentration, et ce, pour un même nombre de spermatozoïdes (tant libres qu'encapsulés).

## Revendications

1. Une composition de matériel séminal porcin destiné à l'insémination artificielle de truies et comprenant des capsules incorporant du matériel séminal porcin, enrobé d'un film d'alginate d'un ion bi ou trivalent, **caractérisé en ce que** la concentration en spermatozoïdes du matériel séminal porcin présent dans les capsules, est comprise entre 900 millions et 2000 millions de spermatozoïdes par millilitre.

2. La composition de matériel séminal porcin selon la revendication 1, comprenant en outre une fraction libre de matériel séminal porcin.

3. La composition selon la revendication 2, **caractérisé en ce que** la concentration de spermatozoïdes présents dans la fraction libre du matériel séminal porcin comporte entre 25 millions et 35 millions de spermatozoïdes par millilitre.

4. La composition selon les revendications 2 ou 3, qui comporte :
- entre 1 et 3 milliards de spermatozoïdes encapsulés et
- entre 1 et 2,5 milliards de spermatozoïdes libres (non encapsulés).

5. La composition selon la revendication 4, qui comporte entre 1,5 et 2,5 milliards de spermatozoïdes encapsulés et entre 1,5 et 2 milliards de spermatozoïdes libres (non encapsulés).

6. La composition selon l'une quelconque des revendications précédentes, où ledit ion bivalent est sélectionné parmi le groupe consistant en ions Ba²⁺ et Ca²⁺.

7. Un procédé d'obtention de la composition selon l'une quelconque des revendications précédentes comprenant les étapes suivantes :
- un matériel séminal porcin récolté est soumis à une ou plusieurs étape(s) d'extraction de plus de 50% du plasma séminal présent dans ce matériel séminal porcin,
- le dit matériel séminal porcin enrichi en spermatozoïdes et additionné entre 2 % et 8 % d'un ion bi ou trivalent mis en suspension dans un diluant approprié et ajouté, goutte à goutte, à une solution d'alginate de sodium pour former des capsules enrobant ledit matériel séminal enrichi en spermatozoïdes,
- les capsules obtenues sont récoltées et
- les capsules récoltées sont optionnellement mélangées avec une fraction libre de matériel séminal porcin.

8. Le procédé selon revendication 7, dans lequel un matériel séminal porcin récolté est soumis à une ou plusieurs étapes(s) d'extraction de plus de 75% du plasma séminal présent dans ce matériel séminal porcin.

9. Le procédé selon la revendication 7 ou 8 où les étape(s) d'extraction du plasma séminal porcin comporte(nt) une ou plusieurs étape(s) de centrifugation du matériel séminal porcin suivie(s) d'élimination du plasma séminal présent dans le surnageant obtenu et de récolte du matériel séminal porcin enrichi en spermatozoïdes.

10. Une trousse d'insémination comportant un ou plusieurs flacon(s) contenant la composition ou les éléments de la composition de matériel séminal porcin selon l'une quelconque des revendications précédentes 1 à 6, un moyen d'insémination apte à effectuer l'insémination artificielle d'une truie par cette composition.

11. La trousse selon la revendication 10 comprenant en outre un flacon contenant un dilueur de la composition.

## Patentansprüche

1. **Porcine Samenmaterial-Zusammensetzung** zur künstlichen Befruchtung von Säuen, die Kapseln aufweist, welche porcines Samenmaterial umfassen, das mit einem Alginatfilm mit bi- bzw. trivalentem Ion umhüllt ist, **dadurch gekennzeichnet, dass** die Spermienkonzentration des in den Kapseln vorhandenen porcinen Samenmaterials zwischen 900 Millionen und 2000 Millionen Spermien pro Milliliter beträgt.

2. Porcine Samenmaterial-Zusammensetzung nach Anspruch 1, ferner enthaltend einen freien Anteil an porcinem Samenmaterial.

3. Zusammensetzung nach Anspruch 2, **dadurch gekennzeichnet, dass** die Konzentration an Spermien, die in dem freien Anteil an porcinem Samenmaterial vorhanden sind, zwischen 25 Millionen und 35 Millionen Spermien pro Milliliter beträgt.

4. Zusammensetzung nach Anspruch 2 oder 3, enthaltend:
- zwischen 1 und 3 Milliarden gekapselte Spermien und
- zwischen 1 und 2,5 Milliarden freie (nicht gekapselte) Spermien.

5. Zusammensetzung nach Anspruch 4, enthaltend zwischen 1,5 und 2,5 Milliarden gekapselte Spermien und zwischen 1,5 und 2 Milliarden freie (nicht gekapselte) Spermien.

6. Zusammensetzung nach einem der vorangehenden Ansprüche, wobei das bivalente Ion ausgewählt ist aus der Gruppe bestehend aus Ba²⁺ und Ca²⁺-Ionen.

7. **Verfahren zum Gewinnen** der Zusammensetzung nach einem der vorigen Ansprüche, umfassend die nachfolgenden Schritte:
- Aussetzen eines gewonnenen porcinen Samenmaterials einem oder mehreren Schritt(en) der Extraktion von über 50% des Samenplasmas, das in diesem porcinen Samenmaterial vorhanden ist,
- Anreichern des porcinen Samenmaterials mit Spermien und Versetzen von zwischen 2% und 8% mit einem bi- bzw. trivalenten Ion, das in einem geeigneten Verdünner suspendiert und tröpfchenweise einer Natriumalginatlösung beigemengt wird, um Kapseln zu bilden, welche das mit Spermien angereicherte Samenmaterial umhüllen,
- Gewinnen der entstandenen Kapseln und
- optional Vermischen der gewonnenen Kapseln mit einem freien Anteil an porcinem Samenmaterial.

8. Verfahren nach Anspruch 7, wobei ein gewonnenes porcines Samenmaterial einem oder mehreren Schritt(en) der Extraktion von über 75% des Samenplasmas ausgesetzt wird, das in diesem porcinen Samenmaterial vorhanden ist.

9. Verfahren nach Anspruch 7 oder 8, wobei der bzw. die Schritt(e) der Extraktion des porcinen Samenplasmas einen oder mehrere Schritt(e) des Zentrifugierens von dem porcinen Samenmaterial, gefolgt von der Entfernung des im entstandenen Überstand vorhandenen Samenplasmas und der Gewinnung des mit Spermien angereicherten porcinen Samenmaterials umfassen.

10. **Inseminationsbesteck** mit einem oder mehreren Röhrchen, welche(s) die Zusammensetzung bzw. die Elemente der porcinen Samenmaterialzusammensetzung nach einem der vorangehenden Ansprüche 1 bis 6 enthält oder enthalten, und mit einer Inseminationseinrichtung, dazu angepasst, die künstliche Befruchtung einer Sau mit dieser Zusammensetzung durchzuführen.

11. Inseminationsbesteck nach Anspruch 10, ferner enthaltend ein Röhrchen, das einen Verdünner für die Zusammensetzung enthält.

## Claims

1. A porcine seminal material composition intended for artificial insemination of sows and comprising capsules incorporating porcine seminal material, coated with a film of an alginate of a bi- or tri-valent ion, **characterized in that** the concentration of spermatozoa of the porcine seminal material present in the capsules, is comprised between 900 million and 2,000 million spermatozoa per milliliter.

2. The porcine seminal material composition according to claim 1, further comprising a free fraction of porcine seminal material.

3. The composition according to claim 2, **characterized in that** the concentration of spermatozoa present in the free fraction of these porcine seminal material include between 25 million and 35 million spermatozoa per milliliter.

4. The composition according to claims 2 or 3, which includes:
- between 1 and 3 thousand million encapsulated spermatozoa and
- between 1 and 2.5 thousand million free spermatozoa (non-encapsulated).

5. The composition according to claim 4, which includes between 1.5 et 2.5 thousand million encapsulated spermatozoa and between 1.5 et 2 thousand million free spermatozoa (non-encapsulated)

6. The composition according to any of the preceding claims, wherein said bivalent ion is selected from the group consisting in Ba²⁺ and Ca²⁺ ions.

7. A method for obtaining the composition according to any of the preceding claims comprising the following steps:
- A collected porcine seminal material is subject to one or several steps for extraction by more than 50% of the seminal plasma present in this porcine seminal material,
- said porcine seminal material enriched with spermatozoa and added with between 2% and 8% of a bi- or tri-valent ion suspended in a suitable diluent and added dropwise to a solution of sodium alginate in order to form capsules coating said seminal material enriched with spermatozoa,
- the obtained capsules are collected and
- the collected capsules are optionally mixed with a free fraction of the porcine seminal material.

8. The method according to claim 7 wherein a collected porcine seminal material is subject to one or several step(s) for extraction by more than 75% of the seminal plasma present in this porcine seminal material

9. The method according to claim 7 or 8 wherein the step(s) for extracting the porcine seminal plasma include(s) one or several steps for centrifugation of the porcine seminal material followed by removal of the seminal plasma present in the obtained supernatant and for collecting the porcine seminal material enriched with spermatozoa.

10. An insemination kit including one or several flasks containing the composition or the elements of the composition of porcine seminal material according to any of the preceding claims 1 to 6, an insemination means able to carry out artificial insemination of a sow with this composition.

11. The insemination kit according to claim 10 further including a flask containing an extender for this composition.
